# EUROPÄISCHE PATENTSCHRIFT

(11) **EP 0 316 599 B1**
(45) Veröffentlichungstag und Bekanntmachung des Hinweises auf die Patenterteilung: **04.05.1994**
(21) Anmeldenummer: 88117388.4
(22) Anmeldetag: 19.10.1988
(51) Int. Cl.: G01N 33/48

(54) **Durchflussvorrichtung für die Verwendung in einer Blutungszeitmesseinrichtung und Verfahren zur Messung der Blutungszeit**
Flow-through apparatus for use in measuring bleeding time, and method for measuring bleeding time
Appareil à écoulement à utiliser pour la mesure de temps de saignement et procédé de mesure du temps de saignement

(30) Priorität: 19.11.1987 DE 3739247
(43) Veröffentlichungstag der Anmeldung: 24.05.1989
(73) Patentinhaber: BAXTER DIAGNOSTICS INC., Deerfield, IL 60015 (US)
(72) Erfinder: Freiherr von der Goltz, Volker, D-83370 Seeon (DE)
(74) Vertreter: von Puttkamer, Nikolaus, Dipl.-Ing. Patentanwälte Haft, von Puttkamer Berngruber, Czybulka

(56) Entgegenhaltungen:
- EP-A- 0 138 190
- DE-A- 3 541 057
- DE-C- 3 247 815

## Beschreibung

Die Erfindung betrifft eine Durchflußvorrichtung für die Verwendung in einer Blutungszeitmeßeinrichtung nach dem Oberbegriff des Anspruchs 1 sowie ein Verfahren zur Messung der Blutungszeit nach dem Oberbegriff des Anspruchs 17.

Bei einer aus der DE-OS 32 47 818 bekannten Vorrichtung zur Messung der Blutungszeit in vitro wird eine Durchflußvorrichtung verwendet, bei der die Trennwand als poröser Teil mit einer Apertur ausgebildet ist. Der poröse Teil liegt auf einer luftundurchlässigen Auflage auf und soll so ausgebildet sein, daß nur durch die Apertur Blut hindurchtritt und nicht seitlich an dem porösen Teil vorbeigelangen kann. Es wird hierbei eine Vorrichtung und ein Verfahren zur Messung der Blutungszeit in vitro gewonnen, bei dem die Blutungsverhältnisse unter In-vivo-Bedingungen nachgebildet werden können.

Aus der DE-OS 35 41 057 ist ein Verfahren bekannt, bei dem das zu untersuchende Blut mit einem bestimmten Sollansaugdruck, der durch Rückkopplung gewonnen wird, in eine Kapillare eingesaugt wird, und bei dem als Maß für die Aggregation bzw. Koagulation der Thrombozyten die Blutflußmenge in der Kapillare bestimmt wird.

Auch hierdurch lassen sich Blutungsverhältnisse unter In-vivo-Bedingungen genau nachbilden.

Aufgabe der Erfindung ist es, eine Vorrichtung und ein Verfahren der eingangs genannten Art zu schaffen, bei dem nicht nur In-vivo-Bedingungen genau nachgebildet sind, sondern auch eine hohe Reproduzierbarkeit der Meßergebnisse erreicht wird.

Diese Aufgabe wird erfindungsgemäß bei der Vorrichtung durch die kennzeichnenden Merkmale des Anspruchs 1 und beim Verfahren durch die kennzeichnenden Merkmale des Anspruchs 17 gelöst.

Durch die Erfindung wird gewährleistet, daß an einer genau definierten Stelle, d. h. an der Apertur, die in einer Trennwand oder einem anderen Bauteil, z. B. einem Gehäuseteilboden, vorgesehen sein kann, die Thrombusbildung stattfindet, und auf diese Weise genau reproduzierbare Meßergebnisse für die Thrombozytenaggregation, insbesondere in der ersten Phase, erreicht werden. Ferner wird eine genaue Nachbildung der In-vivo-Bedingungen der Blutungsverhältnisse erreicht. Die erzielte Varianz der Meßergebnisse liegt unter 5 %, womit die Anforderungen für klinische und wissenschaftliche Anwendungen erfüllt sind.

Die Trennwand kann, wie es aus der deutschen Patentschrift 32 47 815 bekannt ist, als poröser Teil (Porengröße kleiner als 5 µm und größer als 0,01 µm) mit einer Apertur, deren Durchmesser zwischen 50 und 300 µm, bevorzugt 150 und 250 µm, beträgt, ausgebildet sein. Der poröse Teil kann mit Kollagen durchsetzt und beschichtet sein.

Es ist jedoch auch möglich, die Trennwand aus einem nichtporösen Material, z. B. aus Kunststoffplättchen, herzustellen, die insbesondere im Bereich der Apertur mit Kollagen beschichtet sind. Auch ist es möglich, eine Kunststofffolie für die Trennwand zu verwenden, die durch eine Trägerscheibe oder andere Abstützmittel abgestützt ist. Die Kunststoffscheibe kann auch als künstliche Haut, wie sie beispielsweise als Hautimplantat aus der US-PS 4 458 678 bekannt ist, ausgebildet sein. Auch ist es möglich, ein Kunststoffplättchen oder eine Kunststoffolie zu verwenden, das bzw. die aus einer polymerisierten Verbindung auf Acrylsäurebasis besteht, wie sie beispielsweise in der US-PS 4 451 568 beschrieben ist, und an welche das Kollagen kovalent, insbesondere über Oxirangruppen, gebunden ist. Als Trennwandmaterial eignet sich auch Celluloseacetat, das mit Kollagen beschichtet ist. Anstelle von Kollagen kann auch ein anderes Thrombozytenaggregation induzierendes Mittel, z. B. Adenosindiphosphat, oder ein Thrombozyten aktivierendes Mittel, z. B. PAF, das ein Phospholipid (Römpps Chemie-Lexikon, 8. Auflage, S. 3159, 3160) ist, zur Beschichtung zumindest im Bereich der Apertur verwendet werden. Der Durchmesser der Apertur kann 20 µm bis 500 µm, wie der Innendurchmesser des Saugröhrchens, betragen.

Bei der Verwendung der Durchflußvorrichtung ist es von Vorteil, mit einem bestimmten konstanten Ansaugdruck zu arbeiten, mit dem das Blut durch das Ansaugröhrchen und durch die Apertur gesaugt wird, und dabei den zeitlichen Verlauf der Blutflußmenge zu messen. Hierzu kann beispielsweise ein Verfahren nach der DE-OS 35 41 057 zur Anwendung kommen.

Durch Zugabe von die Blutungsverhältnisse beeinflussenden Mitteln in das zu untersuchende Blut können auch bestimmte Diagnosehinweise oder Hinweise auf pharmakologische Beeinflussung des Blutes, z. B. der Thrombozytenadhäsion oder -aggregation, z. B. durch schmerzstillende Mittel, Schlafmittel oder dgl. gewonnen werden. Bei diesen Mitteln kann es sich um gerinnungshemmende Mittel, wie beispielsweise Heparin, Na-Citrat, Kumarinderivate oder um Thrombozytenaggregationshemmer, wie beispielsweise Azetylsalizylsäure, Sulfinpyrazon oder Ticlopidin, oder auch um ein Thrombozytenaggregation induzierendes Mittel, wie beispielsweise Adenosindiphosphat oder Kollagen handeln. Hierbei können auch mehrere Meßvorgänge durchgeführt werden, bei denen ohne oder mit den angegebenen, die Blutungsverhältnisse beeinflussenden Mitteln gearbeitet wird, um dann aus den verschiedenen Meßergebnissen die gewünschten Diagnosehinweise zu gewinnen.

Beispielsweise läßt sich ein Nachweis des v. Willebrand-Syndroms wie folgt erzielen. Dieses Syndrom ist auf eine verringerte Adhäsionsfähigkeit der Blutplättchen zurückzuführen, weshalb bei der Messung eine verzögerte Abnahme der Durchflußmenge feststellbar ist. Wenn man in einem nachfolgenden Meßlauf dem Blut Ristocetinum zugibt, stellt man fest, daß diese verzögerte Abnahme der Durchflußmenge, die aus der Thrombusbildung resultiert, nicht mehr auftritt.

Eine weitere Anwendungsmöglichkeit kann die Vorrichtung bei rheologischen Blutuntersuchungen (Hämorrheologie) bzw. bei der Viskositätsbestimmung von Blut finden. Hierzu können bei Aufrechterhaltung von konstantem Ansaugdruck zu unterschiedlichen Zeitpunkten die jeweiligen Durchflußmengen gemessen werden.

Ferner ist es möglich, auch sich ändernde Drücke für den Bluttransport durch das Saugröhrchen anzuwenden und die jeweiligen Durchflußmengen zu bestimmen. Ferner können pulsierende Drücke für den Bluttransport angewendet werden.

Zum Beispiel lassen sich folgende Diagnosehinweise gewinnen:
Störung der primären Haemostase, prä- bzw. postoperative Überwachung der Thrombozytenfunktion, Testung der Thrombozytenfunktion in Blutkonserven, Messung der Thrombozytenfunktion bei Thrombozytopenien (Leukosen), Messung der Thrombozytenfunktion bei Säuglingen und Kindern, Messen der pharmakologischen Beeinflussung der Thrombozyten-Adhäsion und Aggregation, Messen der Thrombozytenfunktion bei Knochenmarktransplantationen, Prüfung der primären Hämostase nach Brandverletzungen, verminderte Thrombozytenadhäsion, verminderte Thrombozytenaggregation, Thrombozytenfunktion nach Gabe von DDAVP, Viskositätsbestimmung von Blut.

Anhand der beiliegenden Figuren wird an einem Ausführungsbeispiel die Erfindung noch näher erläutert. Es zeigt:
- Fig. 1: schematisch ein Ausführungsbeispiel der Durchflußvorrichtung;
- Fig. 2: einen Teilausschnitt in dieser Durchflußvorrichtung;
- Fig. 3: ein weiteres Ausführungsbeispiel und
- Fig. 4: eine vergrößerte Darstellung des Bereichs um die Apertur bei einem anderen Ausführungsbeispiel.

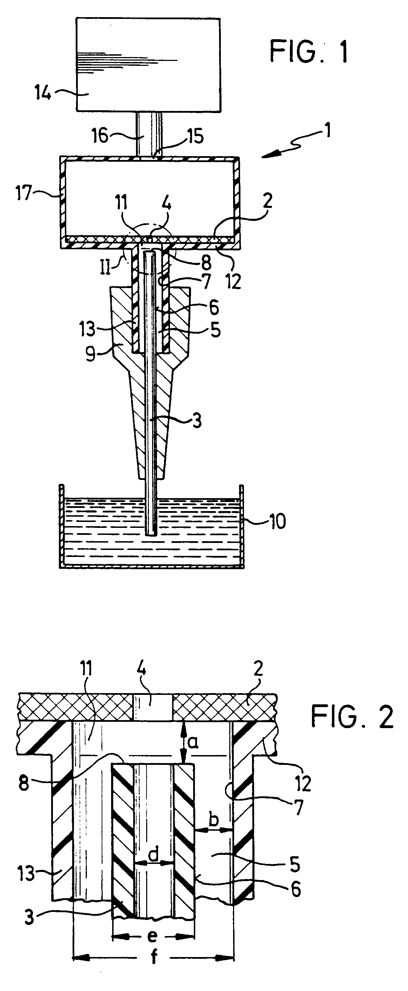

Eine in der Fig. 1 dargestellte Durchflußvorrichtung 1, die ein Ausführungsbeispiel der Erfindung ist, besitzt Gehäuseteile 17 und 9, deren Bestandteile, wie im einzelnen noch erläutert wird, ineinandergesteckt werden können. Der Gehäuseteil 17 ist im wesentlichen als Aufnahmebehälter ausgebildet mit einem Volumen, das etwa dem Volumen entspricht, welches das zu messende Blut in einem Vorratsbehälter 10 einnimmt. Am Boden des Gehäuseteils 17 ist ein Wandteil, z. B. eine Trennwand 2 vorgesehen, die eine Apertur 4 aufweist. Diese Trennwand kann so ausgebildet sein, wie es in der deutschen Patentschrift 32 47 815 beschrieben ist, oder sie kann auch als nichtporöses Kunststoffplättchen ausgebildet sein, das insbesondere im Bereich der Apertur oder ganz mit Kollagen beschichtet ist. Diese Trennwand 2, welche druckdicht ausgebildet ist und an ihrem Rand druckdicht in die Wandung des Gehäuseteils 17 eingesetzt ist, liegt auf dem Boden des Gehäuseteils 17 auf. Zusätzlich kann die Trennwand 2 noch von einer - in der Figur nicht dargestellten - darüber angeordneten Stützeinrichtung, die auf der Oberseite der Trennwand 2 aufliegt, abgestützt sein. Der Boden des Gehäuseteils 17 besitzt eine Öffnung 11, die die Apertur 4 in der Trennwand 2 umgibt. Die Trennwand 2 liegt druckdicht auf dem Boden 12 des Gehäuseteils 17 auf.

Wie insbesondere auch aus der Fig. 2 zu ersehen ist, wird die Öffnung 11 im Boden 12 des Gehäuseteils 17 von einer Innenwand 7 umgeben, die die Innenwand eines an dem Boden 12 angeformten Röhrchens 13 ist. Dieses Röhrchen 13 ist in eine entsprechend ausgebildete Aufnahmebohrung des Gehäuseteils 9 eingesetzt, wie es die Figur 1 zeigt. Der Außendurchmesser des Röhrchens 13 ist dabei an den Innendurchmesser dieser Aufnahmebohrung angepaßt, so daß ein Paßsitz erzielt wird.

Ein Saugröhrchen 3, durch welches das zu untersuchende Blut aus dem Vorratsbehälter 10 gesaugt wird, bildet mit seiner Durchgangsbohrung den haemodynamischen Widerstand. Es ist in eine Durchgangsbohrung des Gehäuseteils 9 fest eingesetzt und ragt in den von der Innenwand 7 des Röhrchens 13 umgebenen Raum. Dabei ist ein Zwischenraum 5 zwischen der Innenwand 7 und einer Mantelfläche 6 des Saugröhrchens 3 vorhanden, durch die ein lockeres Einführen des oberen Teils des Saugröhrchens 3 in den vom Röhrchen 13 umschlossenen Raum gewährleistet wird. Der Abstand der Mantelfläche 6 des Saugröhrchens 3 von der parallel dazu verlaufenden Innenwand 7 kann bis zu 1,0 mm, insbesondere 0,5 mm, betragen. Dieser Abstand ist in der Fig. 2 mit b bezeichnet. Dieser Abstand b gewährleistet unter Berücksichtigung vorhandener Toleranzen ein einwandfreies Einsetzen des Saugröhrchens 3 in das Röhrchen 13. Ferner kann dieser Abstand b so bemessen sein, daß beim Messen kein Blut in den Zwischenraum 5 zwischen der Innenwand 7 des Röhrchens 13 und der Mantelfläche 6 des Saugröhrchens 3 gelangt.

Der Innendurchmesser d des Saugröhrchens 3 kann 20 bis 500 µm und hier vorzugsweise 150 bis 250 µm betragen. Es besteht vorzugsweise aus Polytetrafluorethylen.

Das Saugröhrchen 3 ist mit seinem Röhrchenende 8 bis in die Nähe der Apertur 4 der Trennwand 2 herangeführt. Der Abstand a des Röhrchenendes 8 vom unteren Rand der Apertur 4 beträgt 0,1 bis 6 mm. Der Außendurchmesser e des Saugröhrchens 3 ist so bemessen, daß eine ausreichende mechanische Stabilität gewährleistet ist und kann 0,05 bis 1,5 mm, bevorzugt 1,0 mm, betragen. Der Innendurchmesser f des von der Innenwand 7 umschlossenen Raums hängt aus obengenannten Gründen auch vom Außendurchmesser e des Saugröhrchens 3 ab und kann beim Ausführungsbeispiel 1,5 bis 2,5 mm, bevorzugt 2,0 mm, betragen.

Zum Messen des Blutungsverhaltens des zu untersuchenden Blutes wird mit Hilfe einer Ansaug- und Meßeinrichtung 14 das Blut aus dem Vorratsbehälter 10 durch das Saugröhrchen 3 gesaugt. Hierzu wird ein entsprechender Unterdruck im Innern des Gehäuseteils 17, das über eine Öffnung 15 und ein Saugrohr 16 mit der Ansaug- und Meßeinrichtung 14 verbunden ist, ein Unterdruck aufgebaut. Dieser Unterdruck entsteht auch stromaufwärts von der Apertur 4 in dem von der Innenwand 7 umschlossenen Innenraum, in den das Saugröhrchen 3 ragt. Ferner bildet sich dieser Unterdruck aus in der Durchgangsbohrung (Kapillaren) des Ansaugröhrchens 3. Dies wird dadurch gewährleistet, daß das Röhrchen 13, das druckdicht die Apertur 4 an der Unterseite der Trennwand 2 umgibt, auch druckdicht in die entsprechende Ausnehmung des Gehäuseteils 9 eingesetzt ist.

Die Ansaug- und Meßeinrichtung 14 arbeitet so, daß über die Ansaugleitung 16 bis in die Durchgangsbohrung des Ansaugröhrchens 3 bei der Messung ein konstanter Ansaugdruck aufrecht erhalten wird. Beispielsweise kann die Ansaug- und Meßeinrichtung 14 so ausgebildet sein, wie sie aus der DE-OS 35 41 057 bekannt ist.

Während des Meßvorgangs bildet sich im Bereich der Apertur 4, insbesondere in dem Bereich um die Apertur an der Unterseite der Trennwand 2 und in die Apertur 4 hinein, durch Aggregation der Thrombozyten allmählich ein Pfropfen aus, welcher die Apertur 4 schließlich ganz verschließt. Seitlich in Richtung zur Innenwand 7 hin wegfließendes Blut lagert sich in dem Bereich an, der durch den Abstand a des Röhrchenendes 8 von der Unterseite der Trennwand 2 gebildet wird. Es hat sich herausgestellt, daß bei entsprechender Wahl der Abmessungen gewährleistet ist, daß zwischen die Mantelfläche 6 und den gegenüberliegenden Teil der Innenwand 7 kein Blut eindringt. Ferner hat sich herausgestellt, daß die Thrombusbildung reproduzierbar immer an der Apertur 4 stattfindet. Man gewinnt dadurch eine hohe Reproduzierbarkeit der Meßergebnisse und damit eine genaue Aussage über das Blutungsverhalten, insbesondere über die Thrombozytenaggregation in der ersten Phase.

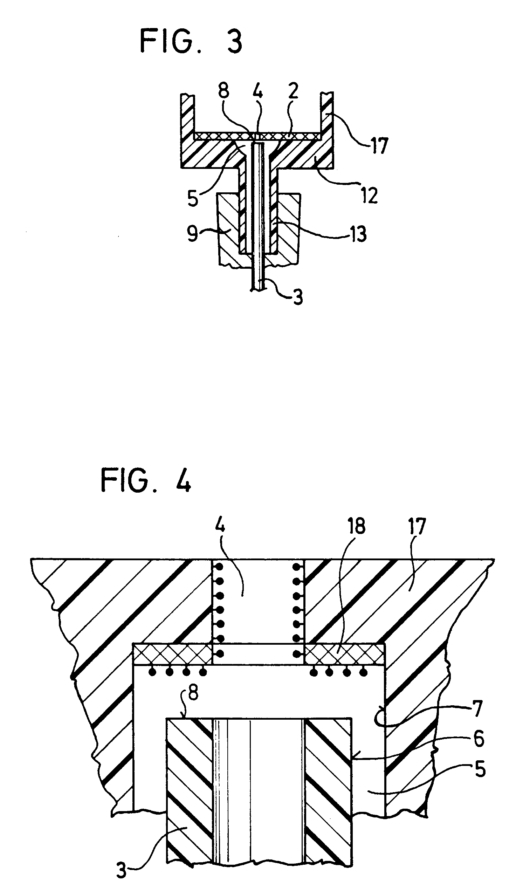

Bei dem in der Fig. 3 dargestellten Ausführungsbeispiel ist der die Apertur 4 umgebende Innenraum 5, in welchen das Saugröhrchen 3 ragt, im Bereich des Röhrchenendes gegenüber dem übrigen Teil erweitert ausgebildet.

Bei den dargestellten Ausführungsbeispielen ist es auch möglich, den Wandteil, den die Trennwand 2 bildet, einstückig mit dem Gehäuseteil 17 auszubilden. Eine derartige Ausführungsform kann beispielsweise so ausgestaltet sein, wie es in Fig. 4 dargestellt ist. Bei dieser Ausführungsform ist die Apertur 4 direkt in den Bodenteil des Gehäuseteils 17 eingeformt. Auch der die Apertur 4 umgebende Innenraum 5, welcher von der Innenwand 7 umgeben wird, ist in den Boden des Gehäuseteils 17 eingeformt bzw. an diesen angeformt. Das Saugröhrchen 3 ragt mit seinem Röhrchenende 8 in diesen eingeformten Innenraum 5, wie es in der Fig. 4 dargestellt ist. Zumindest der um die Apertur 4 liegende, dem Röhrchenende 8 zugewandte Bereich ist mit einer Schicht 18 versehen, die aus einem Thrombozytenaggregation induzierenden Mittel oder einem Thrombozyten aktivierenden Faktor gebildet ist.

Bei der Durchführung der Messung mit der erfindungsgemäßen Durchflußvorrichtung setzen sich in dem Bereich zwischen dem Röhrchenende 8 und der Apertur 4 seitlich von der Apertur Spülmittel, wie z. B. NaCl-Lösung oder Albumin, ab, ohne in den Zwischenraum zwischen der Innenwand 7 und der Mantelfläche 6 zu gelangen. Das nachfolgende Blut fließt durch die Apertur 4, und aufgrund der Adhäsion der Thrombozyten haften Thrombozyten im Bereich der Apertur 4, und es bildet sich ein Thrombus, der zur Verstopfung der Apertur 4 führt.

Das Trennwandmaterial kann aus extrazellulärer Matrix bestehen, die selbst Thrombozytenaggregation induzierende Eigenschaften aufweist oder zusätzlich mit einem Thrombozytenaggregation induzierenden Mittel versehen ist. Das Trennwandmaterial kann auch aus einem Formkörper aus Kollagenmasse bestehen. Ferner eignet sich als Trennwandmaterial auch Tierhaut.

Wenn die Vorrichtung für die oben erwähnten Diagnosehinweise verwendet werden soll, können die die Blutungsverhältnisse beeinflussenden Mittel dem zu untersuchenden Blut auch über das Trennwandmaterial zugeführt werden. Hierzu können die Substanzen, welche in Abhängigkeit von einer bestimmten, bei der Messung durchzuführenden Diagnose ausgewählt werden, auch in das Trennwandmaterial, welches hierzu insbesondere porös ausgebildet ist, eingelagert sein. Die Substanzen werden dann vom Trennwandmaterial während des Meßvorgangs dem Blut Zugeführt.

## Patentansprüche

1. Durchflußvorrichtung für die Verwendung in einer Blutungszeitmeßeinrichtung mit
- einem Durchflußgehäuse, in dessen Innern eine oder mehrere Apertur(en) angeordnet ist bzw. sind, durch die das zu messende Blut fließt;
- einer stromabwärts von der Apertur vorgesehenen Aufnahmeeinrichtung für die durch die Apertur geflossene Blutmenge, und
- einem stromaufwärts von der Apertur angeordneten Saugröhrchen,
dadurch gekennzeichnet, daß
- das Saugröhrchen (3) in einen stromaufwärts von der Apertur (4) gelegenen, die Apertur (4) druckdicht umgebenden Innenraum (5) bis in die Nähe der Apertur (4) ragt und
- ein auf die Apertur (4) gerichtetes Röhrchenende (8) des Saugröhrchens (3) von der Apertur (4) einen solchen Abstand (a) aufweist, daß bei einem bestimmten Ansaugdruck eine Thrombusbildung in der Apertur (4) stattfindet.

2. Durchflußvorrichtung nach Anspruch 1, dadurch gekennzeichnet, daß die Apertur (4) in einer druckdicht ausgebildeten Trennwand (2), die mit der Gehäuseinnenseite druckdicht verbunden ist, vorgesehen ist.

3. Durchflußvorrichtung nach Anspruch 1 oder 2, dadurch gekennzeichnet, daß zumindest der Bereich um die Apertur (4) mit einem Thrombozytenaggregation induzierenden Mittel oder einem Thrombozyten aktivierenden Faktor beschichtet ist.

4. Durchflußvorrichtung nach einem der Ansprüche 1 bis 3, dadurch gekennzeichnet, daß ein Zwischenraum (5) zwischen zumindest einem sich unmittelbar an das Röhrchenende (8) anschließenden Flächenteil der Mantelfläche (6) des Saugröhrchens (3) und einer parallel dazu verlaufenden Innenwand (7) vorhanden ist.

5. Durchflußvorrichtung nach einem der Ansprüche 1 bis 4, dadurch gekennzeichnet, daß das Saugröhrchen (3) einen Innendurchmesser (d) von 20 - 500 µm, insbesondere 150 - 250 µm aufweist.

6. Durchflußvorrichtung nach einem der Ansprüche 1 bis 5, dadurch gekennzeichnet, daß der Abstand (a) des Saugröhrchenendes (8) zur Apertur (4) 0,1 - 6,0 mm, insbesondere 2,0 mm, beträgt.

7. Durchflußvorrichtung nach einem der Ansprüche 1 bis 6, dadurch gekennzeichnet, daß der Abstand (b) zwischen Mantelfläche (6) des Saugröhrchens (3) und parallel verlaufender Innenwand (7) > 0 - 2,0 mm, insbesondere 0,5 mm, beträgt.

8. Durchflußvorrichtung nach einem der Ansprüche 1 bis 7, dadurch gekennzeichnet, daß das Trennwandmaterial aus einem Kunststoff mit bindungsaktiven Gruppen an der Oberfläche besteht, über die Kollagen kovalent gebunden ist.

9. Durchflußvorrichtung nach Anspruch 8, dadurch gekennzeichnet, daß das Trennwandmaterial aus einem oxirangruppenhaltigen Kunststoff besteht.

10. Durchflußvorrichtung nach einem der Ansprüche 1 bis 9, dadurch gekennzeichnet, daß das Saugröhrchen (3) in die die Apertur (4) druckdicht umgebende röhrchenförmig ausgebildete Innenwand (7) einschiebbar ist.

11. Durchflußvorrichtung nach einem der Ansprüche 1 bis 10, dadurch gekennzeichnet, daß das Saugröhrchen (3) in einem Gehäuseteil (9) befestigt ist, der mit einem zweiten, die Innenwand (7) aufweisenden Gehäuseteil (17) verbindbar ist.

12. Durchflußvorrichtung nach einem der Ansprüche 1 bis 11, dadurch gekennzeichnet, daß sie oder Teile davon als Einwegteil(e) ausgebildet ist bzw. sind.

13. Durchflußvorrichtung nach einem der Ansprüche 1 bis 12, dadurch gekennzeichnet, daß das Trennwandmaterial aus extrazellulärer Matrix besteht, die ein Thrombozytenaggregation induzierendes Mittel ist oder mit einem solchen Mittel versehen ist.

14. Durchflußvorrichtung nach einem der Ansprüche 1 bis 13, dadurch gekennzeichnet, daß das Trennwandmaterial ein Formkörper aus Kollagenmasse ist.

15. Durchflußvorrichtung nach einem der Ansprüche 1 bis 13, dadurch gekennzeichnet, daß das Trennwandmaterial aus Tierhaut besteht.

16. Durchflußvorrichtung nach einem der Ansprüche 1 bis 12, dadurch gekennzeichnet, daß das Trennwandmaterial aus einem Thrombozytenaggregation induzierenden Kunststoff besteht.

17. Verfahren zur Messung der Blutungszeit, insbesondere der ersten Phase der Thrombozytenaggregation, bei dem das zu untersuchende Blut durch das Saugröhrchen mit einem bestimmten Ansaugdruck gesaugt und die durch das Saugröhrchen geflossene Blutmenge gemessen wird, dadurch gekennzeichnet, daß unter Verwendung einer Durchflußvorrichtung nach einem der Ansprüche 1 bis 12 an der Apertur eine Thrombusbildung erzeugt wird.

18. Verfahren nach Anspruch 17, bei welchem dem zu untersuchenden Blut vor dem Einsaugen in das Saugröhrchen bestimmte Substanzen zugeführt werden, welche im Blut ablaufende Vorgänge beeinflussen, dadurch gekennzeichnet, daß die jeweilige, dem Blut zugeführte Substanz in Abhängigkeit von einer bestimmten, bei der Messung durchzuführenden Diagnose ausgewählt wird.

19. Verfahren nach Anspruch 17, bei welchem dem zu untersuchenden Blut bestimmte Substanzen zugeführt werden, welche im Blut ablaufende Vorgänge beeinflussen, dadurch gekennzeichnet, daß die jeweilige dem Blut zuzuführende Substanz, die in Abhängigkeit von einer bestimmten, bei der Messung durchzuführenden Diagnose ausgewählt ist, vom Trennwandmaterial dem Blut zugeführt wird.

20. Verfahren nach Anspruch 18 oder 19, dadurch gekennzeichnet, daß gerinnungshemmende Mittel (Antikoagulantien, z. B. Heparin, Na-Citrat, Kumarinderivate) dem Blut zugeführt Werden.

21. Verfahren nach Anspruch 18 oder 19, dadurch gekennzeichnet, daß Thrombozytenaggregationshemmer (z. B. Azetylsalizylsäure, Sulfinpyrazon, Ticlopidin) dem Blut zugeführt werden.

22. Verfahren nach Anspruch 18 oder 19, dadurch gekennzeichnet, daß ein Thrombozytenaggregation induzierendes Mittel (z. B. Adenosindiphosphat, Kollagen, PAF, Ristocetin) dem Blut zugeführt wird.

23. Verwendung eines oder mehrerer der in den Ansprüchen 17 bis 22 angegebenen Verfahren für Diagnosezwecke.

24. Verfahren zur rheologischen Blutuntersuchung, gekennzeichnet durch die Verwendung einer Vorrichtung nach einem der Ansprüche 1 bis 16.

25. Verfahren nach Anspruch 24, dadurch gekennzeichnet, daß die Vorrichtung nach einem der Ansprüche 1 bis 16 zur Viskositätsbestimmung von Blut verwendet wird.

26. Verfahren nach Anspruch 24 oder 25, dadurch gekennzeichnet, daß bei Aufrechterhaltung von konstantem Ansaugdruck an unterschiedlichen Zeitpunkten die jeweiligen Durchflußmengen gemessen werden.

27. Verfahren nach Anspruch 24 oder 25, dadurch gekennzeichnet, daß sich ändernde Drücke für den Bluttransport durch das Saugröhrchen angewendet werden.

28. Verfahren nach Anspruch 27, dadurch gekennzeichnet, daß pulsierende Drücke für den Bluttransport durch das Röhrchen verwendet werden.

## Claims

1. A flow device for use in a bleeding time measuring instrument having
- a flow housing, inside which one or more aperture(s) is(are) disposed, through which the blood to be measured flows;
- a receptacle provided downstream from the aperture for the volume of blood which has flowed through the aperture, and
- a suction tube disposed upstream from the aperture,
**characterised in that**
- the suction tube (3) protrudes into an interior space (5), which is situated upstream from the aperture (4) and surrounds the aperture (4) in a pressure-tight manner, right into the vicinity of the aperture and
- a tube end (8) of the suction tube (3) directed towards the aperture (4) comprises such a distance (a) from the aperture (4) that a thrombosis occurs in the aperture (4) at a certain suction pressure.

2. A flow device according to Claim 1,
**characterised in that** the aperture (4) is provided in a pressure-sealed partition (2), which is connected in a pressure-tight manner to the inside of the housing.

3. A flow device according to Claim 1 or 2,
**characterised in that** at least the region around the aperture (4) is coated with a medium inducing platelet aggregation or a platelet-activating factor.

4. A flow device according to one of Claims 1 to 3,
**characterised in that** there is a space (5) between at least one surface part of the shell surface (6) of the suction tube (3) directly connected to the tube end (8) and an inner wall (7) extending parallel thereto.

5. A flow device according to one of Claims 1 to 4,
**characterised in that** the suction tube (3) has an internal diameter (d) of 20 - 500 µm, especially 150 - 250 µm.

6. A flow device according to one of Claims 1 to 5,
**characterised in that** the distance (a) of the suction tube end (8) to the aperture (4) is 0.1 - 6.0 mm, especially 2.0 mm.

7. A flow device according to one of Claims 1 to 6,
**characterised in that** the distance (b) between the shell surface (6) of the suction tube (3) and inner wall (7) extending parallel thereto is > 0 - 2.0 mm, especially 0.5 mm.

8. A flow device according to one of Claims 1 to 7,
**characterised in that** the material of the partition is made from a plastic having bonding-active groups at the surface, via which collagen is covalently bonded.

9. A flow device according to Claim 8,
**characterised in that** the material of the partition is made from a plastic containing oxirane groups.

10. A flow device according to one of Claims 1 to 9,
**characterised in that** the suction tube (3) can be inserted into the inner wall (7) constructed in a tubular shape and surrounding the aperture (4) in a pressure-tight manner.

11. A flow device according to one of Claims 1 to 10,
**characterised in that** the suction tube (3) is attached in a housing part (9), which can be connected to a second housing part (17) comprising the inner wall (7).

12. A flow device according to one of Claims 1 to 11,
**characterised in that** it or parts thereof is/are constructed as a disposable part/disposable parts.

13. A flow device according to one of Claims 1 to 12,
**characterised in that** the material of the partition is made from extracellular matrix, which is a medium inducing platelet aggregation or is provided with such a medium.

14. A flow device according to one of Claims 1 to 13,
**characterised in that** material of the partition is a member made from collagen compound.

15. A flow device according to one of Claims 1 to 13,
**characterised in that** the material of the partition is made from animal skin.

16. A flow device according to one of Claims 1 to 12,
**characterised in that** the material of the partition is made from a plastic inducing platelet aggregation.

17. A method for measuring bleeding time, in particular the first phase of platelet aggregation, in which the blood to be tested is sucked through the suction tube at a determined suction pressure and the amount of blood which has flowed through the suction tube is measured,
**characterised in that** a thrombosis is produced at the aperture by using a flow device as specified in one of Claims 1 to 12.

18. A method according to Claim 17, in which determined substances, which influence processes occurring in the blood, are supplied to the blood to be tested before it is sucked into the suction tube, **characterised in that** each substance to be supplied to the blood is selected as a function of a determined diagnosis to be performed during measurement.

19. A method according to Claim 17, in which determined substances, which influence processes occurring in the blood, are supplied to the blood to be tested, **characterised in that** each substance to be supplied to the blood, which is selected as a function of a determined diagnosis to be performed during the measurement, is supplied to the blood from the material of the partition.

20. A method according to Claim 18 or 19,
**characterised in that** anti-coagulant agents (anti-coagulants, e.g. heparin, Na citrate, coumarin derivatives) are supplied to the blood.

21. A method according to Claim 18 or 19,
**characterised in that** platelet aggregation inhibitors (e.g. acetylsalicylic acid, sulphinpyrazone, ticlopidin) are supplied to the blood.

22. A method according to Claim 18 or 19,
**characterised in that** a medium inducing platelet aggregation (e.g. adenosine diphosphate, collagen, PAF, ristocetin) is supplied to the blood.

23. Use of one or more of the methods specified in Claims 17 to 22 for diagnostic purposes.

24. A method for rheological blood testing,
**characterised by** the use of a device as specified in one of Claims 1 to 16.

25. A method according to Claim 24,
**characterised in that** the device according to one of Claims 1 to 16 is used to determine the viscosity of blood.

26. A method according to Claim 24 or 25,
**characterised in that** the respective flow rates are measured whilst maintaining constant suction pressure at different times.

27. A method according to Claim 24 or 25,
**characterised in that** changing pressures are used for the transport of blood through the suction tube.

28. A method according to Claim 27,
**characterised in that** pulsating pressures are used for the transport of blood through the tube.

## Revendications

1. Dispositif d'écoulement destiné à être utilisé dans un dispositif de mesure du temps de coagulation comportant
- un coprs à l'intérieur duquel est ou sont disposée(s) une ou plusieurs ouvertures par lesquelles s'écoule le sang à mesurer,
- un dispositif récepteur pour le sang qui s'est écoulé à travers l'ouverture, lequel dispositif est disposé en aval de l'ouverture et
- un petit tube d'aspiration disposé en amont de l'ouverture, caractérisé par le fait
- que le tube d'aspiration (3) pénètre jusqu'au voisinage de l'ouverture (4) dans une chambre (5) intérieure qui est située en amont de l'ouverture (4) et entoure cette dernière (4) de manière étanche à la pression et
- qu'une extrémité (8) du tube d'aspiration (3) tournée vers l'ouverture (4) est disposée à une distance (a) de ladite ouverture (4) telle que, pour une pression d'aspiration déterminée, il se forme un thrombus dans l'ouverture (4).

2. Dispositif d'écoulement selon la revendication 1, caractérisé par le fait que l'ouverture (4) est prévue dans une paroi de séparation (2) étanche à la pression qui est reliée de manière étanche à la pression avec l'intérieur du corps.

3. Dispositif d'écoulement selon la revendication 1 ou 2, caractérisé par le fait qu'au moins la zone située autour de l'ouverture (4) est revêtue d'un agent induisant l'agrégation des thrombocytes ou d'un facteur activant les thrombocytes.

4. Dispositif d'écoulement selon l'une des revendications 1 à 3, caractérisé par le fait qu'il est prévu une chambre intermédiaire (5) entre au moins une partie de la surface périphérique (6) du tube d'aspiration (3) contigue à l'extrémité (8) dudit tube et une paroi intérieure (7) parallèle à celle-ci .

5. Dispositif d'écoulement selon l'une des revendications 1 à 4, caractérisé par le fait que le tube d'aspiration (3) a un diamètre intérieur (d) de 20 - 500 µm, en particulier de 150 - 250 µm.

6. Dispositif d'écoulement selon l'une des revendications 1 à 5, caractérisé par le fait que la distance (a) entre l'extrémité (8) du tube d'aspiration et l'ouverture (4) est de 0,1 - 6,0 mm, en particulier de 2,0 mm.

7. Dispositif d'écoulement selon l'une des revendications 1 à 6, caractérisé par le fait que la distance (b) entre la surface périphérique (6) du tube d'aspiration (3) et la paroi intérieure (7) parallèle à celle-ci est > 0 - 2,0 mm, en particulier de 0,5 mm.

8. Dispositif d'écoulement selon l'une des revendications 1 à 7, caractérisé par le fait que le matériau de la paroi de séparation est une matière synthétique comportant à sa surface des groupes présentant un pouvoir de liaison, par l'intermédiaire desquels le collagène est lié par covalence.

9. Dispositif d'écoulement selon la revendication 8, caractérisé par le fait que le matériau de la paroi de séparation est une matière synthétique contenant des groupes oxirane.

10. Dispositif d'écoulement selon l'une des revendications 1 à 9, caractérisé par le fait que le tube d'aspiration (3) peut être engagé dans la paroi intérieure (7) agencée en forme de petit tube qui entoure de manière étanche à la pression l'ouverture (4).

11. Dispositif d'écoulement selon l'une des revendications 1 à 10, caractérisé par le fait que le tube d'aspiration (3) est fixé dans une partie de corps (9) qui peut être connectée à une deuxième partie de corps (17) comportant la paroi intérieure (7).

12. Dispositif d'écoulement selon l'une des revendications 1 à 11, caractérisé par le fait qu'il est agencé ou que des parties de celui-ci sont agencées sous forme d'élément(s) jetable(s).

13. Dispositif d'écoulement selon l'une des revendications 1 à 12, caractérisé par le fait que le matériau de la paroi de séparation est constitué d'une matrice extracellulaire qui est un agent induisant l'agrégation des thrombocytes ou est pourvue d'un agent de ce type.

14. Dispositif d'écoulement selon l'une des revendications 1 à 13, caractérisé par le fait que le matériau de la paroi de séparation est un corps moulé formé d'une masse de collagène.

15. Dispositif d'écoulement selon l'une des revendications 1 à 13, caractérisé par le fait que le matériau de la paroi de séparation est constitué de peau animale.

16. Dispositif d'écoulement selon l'une des revendications 1 à 12, caractérisé par le fait que le matériau de la paroi de séparation est constitué d'une matière synthétique induisant l'agrégation des thrombocytes.

17. Procédé de mesure du temps de coagulation, en particulier de la première phase de l'agrégation des thrombocytes, dans lequel on aspire le sang à examiner avec une pression d'aspiration déterminée à travers le tube d'aspiration et on mesure la quantité de sang qui s'est écoulée à travers ledit tube , caractérisé par le fait que l'on provoque la formation de thrombus en utilisant un dispositif d'écoulement selon l'une des revendications 1 à 12.

18. Procédé selon la revendication 17, dans lequel on ajoute certaines substances au sang à examiner avant son aspiration à travers le tube d'aspiration, lesquelles substances influent sur les réactions qui se déroulent dans le sang, caractérisé par le fait que la substance ajoutée au sang est choisie en fonction d'un diagnostic déterminé à faire lors de la mesure.

19. Procédé selon la revendication 17, dans lequel on ajoute certaines substances au sang à examiner, lesquelles substances influent sur les réactions qui se déroulent dans le sang, caractérisé par le fait que la substance qui est ajoutée au sang en fonction d'un diagnostic déterminé à faire lors de la mesure est introduite dans le sang par le matériau de la paroi de séparation.

20. Procédé selon la revendication 18 ou 19, caractérisé par le fait que l'on ajoute au sang des agents inhibiteurs de coagulation (anticoagulants, par exemple héparine, citrate de sodium, dérivés de coumarine).

21. Procédé selon la revendication 18 ou 19, caractérisé par le fait que l'on ajoute au sang des agents inhibiteurs de l'agrégation des thrombocytes (par exemple acide acétylsalicylique, sulfinylpyrazone, ticlopidine).

22. Procédé selon la revendication 18 ou 19, caractérisé par le fait que l'on ajoute au sang un agent induisant l'agrégation des thrombocytes (par exemple diphosphate d'adénosine, collagène, PAF, ristocétine).

23. Utilisation d'un ou plusieurs des procédés indiqués dans les revendication 17 à 22 à des fins de diagnostic.

24. Procédé pour l'examen rhéologique du sang, caractérisé par l'utilisation d'un dispositif selon l'une des revendications 1 à 16.

25. Procédé selon la revendication 24, caractérisé par le fait que l'on utilise le dispositif selon l'une des revendications 1 à 16 pour déterminer la viscosité du sang.

26. Procédé selon la revendication 24 ou 25, caractérisé par le fait que la pression d'aspiration étant maintenue constante, on mesure les débits de sang à différents instants.

27. Procédé selon la revendication 24 ou 25, caractérisé par le fait que l'on utilise pour le transport du sang à travers le tube d'aspiration des pressions qui varient.

28. Procédé selon la revendication 27, caractérisé par le fait que l'on utilise pour le transport du sang à travers le tube d'aspiration des pressions pulsatoires.
